(19) Europäisches Patentamt
European Patent Office
Office européen des brevets

(11) Veröffentlichungsnummer: **0 117 531**
**B1**

(12) # EUROPÄISCHE PATENTSCHRIFT

(45) Veröffentlichungstag der Patentschrift: **17.09.86**

(21) Anmeldenummer: **84101894.8**

(22) Anmeldetag: **23.02.84**

(51) Int. Cl.⁴: **C 07 D 295/06,** A 61 K 31/495 // C07C87/60, C07C101/16, C07D241/08, C07D265/32

(54) **N-(3-Trifluormethylphenyl)-N'-propargylpiperazin, seine Herstellung und Verwendung.**

(30) Priorität: **28.02.83 DE 3306964**

(43) Veröffentlichungstag der Anmeldung: **05.09.84 Patentblatt 84/36**

(45) Bekanntmachung des Hinweises auf die Patenterteilung: **17.09.86 Patentblatt 86/38**

(84) Benannte Vertragsstaaten: **AT BE CH DE FR IT LI LU NL SE**

(56) Entgegenhaltungen:
**BE - A - 682 342**
**FR - A - 2 187 311**
**GB - A - 2 078 746**
**US - A - 4 180 574**

**Die Akte enthält technische Angaben, die nach dem Eingang der Anmeldung eingereicht wurden und die nicht in dieser Patentschrift enthalten sind.**

(73) Patentinhaber: **BOEHRINGER INGELHEIM KG, D-6507 Ingelheim am Rhein (DE)**

(72) Erfinder: **Banholzer, Rolf, Dr., Johann-Calvin-Strasse 11, D-6507 Ingelheim (DE)**
Erfinder: **Merz, Herbert, Dr., Rotweinstrasse 53, D-6507 Ingelheim (DE)**
Erfinder: **Stockhaus, Klaus, Dr., Pfarrer-Heberer-Strasse 35, D-6530 Bingen (DE)**
Erfinder: **Jennewein, Hans Michael, Prof. Dr., Idsteiner Strasse 14, D-6200 Wiesbaden (DE)**

**EP 0 117 531 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft die neue Verbindung N-(3--Trifluormethylphenyl)-N'-propargyl-piperazin der Formel I

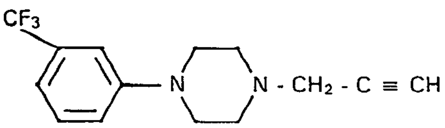

(I)

sowie deren physiologisch verträgliche Säureadditionssalze, Verfahren zu ihrer Herstellung und ihre Verwendung in Pharmazeutika.

Die neue Verbindung kann hergestellt werden, indem man entweder den Piperazinring aufbaut oder in das fertige N-3-Trifluormethylphenylpiperazin den Propargyl-Rest einführt bzw. durch Abwandlung herstellt.

Hierdurch ergeben sich folgende Verfahren:

a) Umsetzung einer Verbindung der allgemeinen Formel II

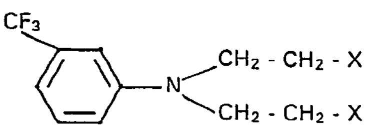

(II)

worin X eine leicht anionisch abspaltbare Gruppe wie ein Halogenatom oder eine Alkyl- bzw. Arylsulfonyloxygruppe bedeutet, mit N-Propargylamin der Formel III

$HC \equiv C - CH_2 - NH_2$ (III)

bei Temperaturen zwischen Raumtemperatur und der Siedetemperatur des Reaktionsgemisches.

b) Umsetzung einer Verbindung der allgemeinen Formel IV

$HC \equiv C - CH_2 - N(CH_2 - CH_2 - X)(CH_2 - CH_2 - X)$ (IV)

worin X die obengenannten Bedeutungen hat, mit 3-Trifluormethylanilin der Formel V

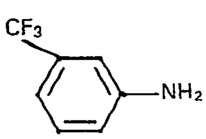

(V)

bei Temperaturen zwischen Raumtemperatur und der Siedetemperatur des Reaktionsgemisches.

c) Umsetzung einer Verbindung der Formel VI

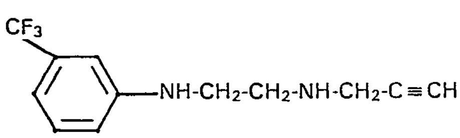

(VI)

mit einer Verbindung der allgemeinen Formel VII

$X - CH_2 - CH_2 - X$ (VII)

worin X die obengenannten Bedeutungen hat, bei Temperaturen zwischen Raumtemperatur und der Siedetemperatur des Reaktionsgemisches.

d) Einführung der Propargylgruppe in die Verbindung N-(3-Trifluormethylphenyl)-piperazin mittels üblicher Propargylierungsmethoden, wie etwa durch Behandlung mittels einer Verbindung der allgemeinen Formel VIII

$HC \equiv C - CH_2 - X$ (VIII)

worin X die oben angegebene Bedeutung hat, bei Temperaturen zwischen Raumtemperatur und der Siedetemperatur des Reaktionsgemisches.

e) Reduktion des Dions der Formel IX

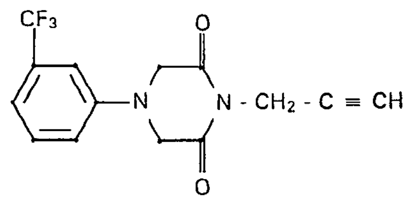

(XI)

mit reduzierenden Hydriden bei Temperaturen zwischen ca. 0°C und der Siedetemperatur des Reaktionsgemisches.

f) Reduktion des Dions der Formel X

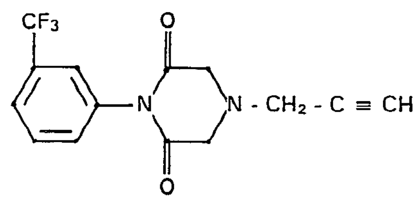

(X)

mit reduzierenden Hydriden bei Temperaturen zwischen ca. 0°C und der Siedetemperatur des Reaktionsgemisches.

g) Reduktion des Dions der Formel XI

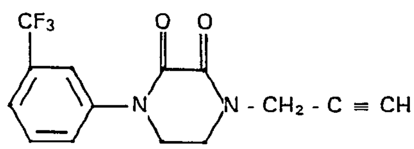

(XI),

mit reduzierenden Hydriden bei Temperaturen zwischen ca. 0°C und der Siedetemperatur des Reaktionsgemisches.

Die vorstehenden Verfahren werden in an sich bekannter Weise durchgeführt.

Man verwendet zweckmässig ein unter den Reaktionsbedingungen inertes Lösungsmittel als Reaktionsmedium, beispielsweise für die Verfahren a) und b) einen niederen aliphatischen Alkohol, etwa Butanol, für das Verfahren c) beispielsweise Toluol. Für das Verfahren d) hat sich Acetonitril als Lösungsmittel bewährt, während für die Verfahren e), f) und g) besonders Ether, etwa Diethylether oder Tetrahydrofuran, benutzt werden können.

Wird bei den Umsetzungen Säure freigesetzt, können Zusätze zum Abfangen der Säure gemacht werden. Hierzu kommen beispielsweise Natriumhydrogencarbonat oder Natriumcarbonat [Verfahren a), b), d) oder organische Basen, etwa Triethylamin (insbesondere bei Verfahren c)], in Betracht.

Als Reduktionsmittel für die Verfahren e), f) und g) eignen sich Hydride wie Lithiumaluminiumhydrid oder Borwasserstoff.

Die Ausgangsverbindungen der allgemeinen Formeln II bis XI sind entweder bereits bekannt oder sie lassen sich leicht nach üblichen Methoden herstellen. Hierzu wird auf die in der DE-PS 2 442 158 näher beschriebenen Methoden verwiesen. Die für das Verfahren d) benutzte Ausgangsverbindung N-(3--Trifluormethylphenyl)-piperazin lässt sich nach der Methode des irischen Patents 28 880 herstellen.

Die Ausgangsstoffe für die Verfahren e) bis g) können gemäss dem folgenden Schema erhalten werden:

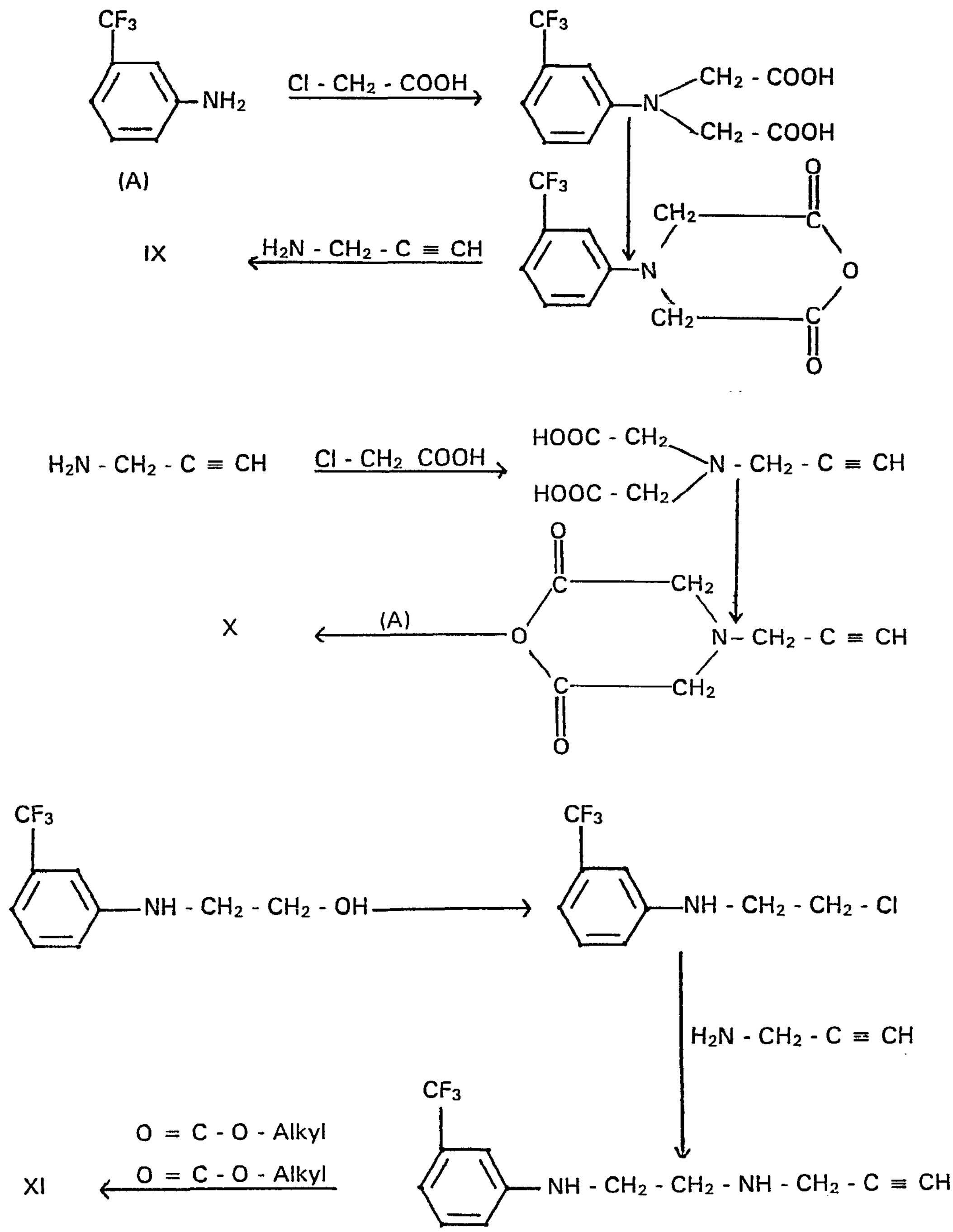


Das erfindungsgemässe N-Arylpiperazin der Formel I kann in üblicher Weise in seine physiologisch verträglichen Säureadditionssalze überführt werden.

Ein geeignetes Säureadditionssalz der erfindungsgemässen Verbindung ist beispielsweise ein Salz, das durch Umsetzung mit einer anorganischen Säure erhalten werden kann, wie z.B. ein Hydrochlorid, Hydrobromid, Phosphat oder Sulfat; oder auch ein Salz, das durch Umsetzung mit einer organischen Säure erhalten werden kann, wie z.B. ein Methansulfonat, Maleinat, Acetat, Oxalat, Lactat, Tartrat,

8-Chlortheophyllinat, Salicylat, Citrat, β-Naphthoat, Adipat, 1,1-Methylen-bis-(2-hydroxy-3-naphthoat) oder ein Salz, das sich von einem sauren synthetischen Harz ableitet wie z.B. von einem sulfonierten Polystyrolharz.

Die Verbindung der Formel I bzw. ihre physiologisch verträglichen Säureadditionssalze zeigen im Tierversuch wertvolle therapeutische, insbesondere analgetische Eigenschaften. Sie zeichnet sich gegenüber bekannten analgetischen Substanzen wie z.B. dem Metamizol-Natrium durch ein weitgehendes Fehlen von Nebenwirkungen aus.

Die im folgenden aufgeführten pharmakologischen Daten zeigen die wertvolle Eigenschaften des erfindungsgemässen N-(3-Trifluormethylphenyl)-N'-propargylpiperazinhydrochlorids im Vergleich zu dem bekannten Analgetikum Metamizol (Novalgin® ).

| Verbindung | Writhing-Test $ED_{50}$ sc/po (mg/kg) | Gait-Test (s.c.) $ED_{50}$ (mg/kg) | Kaninchen-Zahnpulpa-Test (s.c.) $ED_{1V}$ |
|---|---|---|---|
| A. Erfindung N-(3-Trifluormethylphenyl)-N'-propargylpiperazin · HCl | 5,3/43 | 2,8 | ca. 10 |
| B. Vergleich Metamizol-Natrium (Novalgin® ) | 25/80 | 97 | ca. 560 |

*Literatur*

1. Writhing-Test
   Linné, Ph., J. Pharmacol. (Paris) 3,4, p 513-515 (1972)
2. Gait-Test
   Atkinson, D.C. and Cowan, A.J. Pharm. Pharmacol. 26, 727-728 (1974)
3. Kaninchen-Zahnpulpa-Test
   Hoffmeister, F., Arzneimittelf. 16 (Beiheft), 1968.

Die Einzeldosis der erfindungsgemässen Substanzen liegt bei 50 bis 500 mg, vorzugsweise 75 bis 200 mg (oral) bzw. 15 bis 150 mg, vorzugsweise 25 bis 75 mg (parenteral).

Der erfindungsgemässe Wirkstoff bzw. seine Salze können in die üblichen galenischen Anwendungsformen, wie Tabletten, Dragées, Lösungen, Emulsionen, Pulver, Kapseln oder Depotformen gebracht werden, wobei zu deren Herstellung die üblichen pharmazeutischen Hilfsstoffe sowie die üblichen Fertigungsmethoden herangezogen werden können. Entsprechende Tabletten können beispielsweise durch Mischen der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung eines Depoteffekts, wie Carboxypolymethylen, Carboxymethylcellulose, Celluloseacetatphthalat, oder Polyvinylacetat erhalten werden.

Die Tabletten können auch aus mehreren Schichten bestehen. Entsprechend können Dragées durch Überziehen von analog den Tabletten hergestellten Kernen mit üblicherweise in Dragéeüberzügen verwendeten Mitteln, beispielsweise Kollidon oder Schellack, Gummi arabicum, Talk, Titandioxid oder Zucker, hergestellt werden. Zur Erzielung eines Depoteffekts oder zur Vermeidung von Inkompatibilitäten kann der Kern auch aus mehreren Schichten bestehen. Desgleichen kann auch die Dragéehülle zur Erzielung eines Depoteffekts aus mehreren Schichten bestehen, wobei die oben bei den Tabletten erwähnten Hilfsstoffe verwendet werden können.

Säfte des erfindungsgemässen Wirkstoffes bzw. Wirkstoffkombinationen können zusätzlich noch ein Süssungsmittel, wie Saccharin, Cyclamat, Glycerin oder Zucker, sowie ein geschmackverbesserndes Mittel, z.B. Aromastoffe, wie Vanillin oder Orangenextrakt, enthalten.

Sie können ausserdem Suspendierhilfsstoffe oder Dickungsmittel, wie Natriumcarboxymethyl-Cellulose, Netzmittel, beispielsweise Kondensationsprodukte von Fettalkoholen mit Äthylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Komplexonen, hergestellt und in Injektionsflaschen oder Ampullen abgefüllt.

Den Wirkstoff bzw. Wirkstoffkombinationen enthaltende Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen des Wirkstoffes bzw. der Wirkstoffkombinationen mit üblichen Trägermitteln, wie Neutralfetten oder Polyäthylenglykol bzw. dessen Derivaten, herstellen.

Die erfindungsgemässen Substanzen sind auch für die Kombination mit anderen pharmakodynamisch wirksamen Stoffen, wie z.B. Tranquilizern, geeignet.

Die folgenden Beispiele erläutern die Erfindung, ohne sie zu beschränken:

*Beispiel 1*

*N-(3-Trifluormethylphenyl)-N'-propargylpiperazin-hydrochlorid (nach Verfahren a)*

Zu 28,6 g (0,1 mol) N,N-Bis-(2-chlorethyl)-3-(trifluormethyl)-anilin in n-Butanol werden 16,5 g (0,3 mol) Propargylamin allmählich bei Raumtemperatur zugetropft und die Reaktion durch Erhitzen bis auf 100°C zu Ende geführt unter Kontrolle durch Chromatographie. Nach beendeter Reaktion wird das n-Butanol unter vermindertem Druck abdestilliert.

Der Destillationsrückstand wird in Wasser mit der äquivalenten Menge Natriumcarbonat alkalisch gemacht und das N-Propargyl-N'-(3-trifluormethyl-phenyl)-piperazin mit Ether extrahiert. Nach dem

Trocknen mit Natriumsulfat wird unter vermindertem Druck eingedampft und die Base auf üblichem Weg zum Hydrochlorid umgesetzt unter Verwendung einer stöchiometrischen Menge Chlorwasserstoffs. Bereits kleine Mengen von Dihydrochlorid beeinflussen den Schmelzpunkt.

Weisse Kristalle aus Acetonitril, Schmp. 218-219°C (Zers.). Analog lässt sich diese Umsetzung mit N,N-Bis-(2-methyl-sulfonyloxi-ethyl)-3-(trifluormethyl)-anilin durchführen, hergestellt nach Mertes M.P. u.a. J. Med. Chem. 12 (5), 825-28 (1969).

*Beispiel 2*

*N-(3-Trifluormethylphenyl)-N'-propargylpiperazin-hydrochlorid (nach Verfahren b)*

Ein Gemisch aus 16,1 g (0,1 mol) 3-(Trifluormethyl)-anilin und 21,6 g (0,1 mol) N,N-Bis-(2-chlorethyl)-N-propargylamin-hydrochlorid in 150 ml n-Butanol wird unter Rückfluss erhitzt. Die im Verlauf der Reaktion auftretende saure Reaktion wird mit Natriumhydrogencarbonat abgepuffert. Das dabei entstehende Wasser wird azeotrop entfernt. Die über Chromatographie kontrollierte Umsetzung kann einen Überschuss von 3-(Trifluormethyl)-anilin erforderlich machen.

Nach beendeter Reaktion wird die Suspension auf 10°C abgekühlt und das feste Produkt abgesaugt. Das n-Butanol wird unter vermindertem Druck abdestilliert, das abgesaugte Produkt und der Destillationsrückstand in Wasser gelöst und mehrmals mit Ether extrahiert. Die Wasserphase wird mit der äquivalenten Menge Natriumcarbonat alkalisch gemacht und das Reaktionsprodukt mit Ether extrahiert. Nach dem Trocknen mit Natriumsulfat wird unter vermindertem Druck eingedampft und die zurückbleibende Base auf üblichem Weg mit der stöchiometrischen Menge Chlorwasserstoff zum Hydrochlorid umgesetzt.

Weisse Kristalle aus Acetonitril, Schmp. 218-219°C (Zers.).

*Beispiel 3*

*N-(3-Trifluormethylphenyl)-N'-propargylpiperazin-hydrochlorid (nach Verfahren c)*

3.1. *N-(2-Chlorethyl)-3-(trifluormethyl)-anilin-hydrochlorid*

20,5 g (0,1 mol) N-(2-Hydroxiethyl)-3-(trifluormethyl)-anilin werden in 100 ml absolutem Methylenchlorid aufgenommen und auf 0°C abgekühlt. Bei dieser Temperatur werden 14,3 g (0,12 mol) Thionylchlorid zugetropft. Man lässt die Umsetzung unter Rühren auf Raumtemperatur kommen und erhitzt so lange unter Rückfluss unter chromatographischer Kontrolle, bis die Umsetzung beendet ist. Nach Entfernen des Lösungsmittels und des restlichen Thionylchlorids wird das so erhaltene Rohprodukt zur weiteren Umsetzung eingesetzt.

3.2. *N-Propargyl-N'-(3-trifluormethyl-phenyl)-ethylendiamin*

16,5 g (0,3 mol) Propargylamin werden in 100 ml absolutem Methylenchlorid gelöst und bei Raumtemperatur anteilweise mit dem in der Vorstufe erhaltenen N-(2-Chlorethyl)-3-(trifluormethyl)-anilin-hydrochlorid versetzt. Unter chromatographischer Kontrolle wird so lange erhitzt, bis die Reaktion beendet ist.

Nach beendeter Umsetzung wird die Reaktionslösung mit der berechneten Menge Natriumcarbonatlösung extrahiert. Nach dem Trocknen mit Natriumsulfat wird unter vermindertem Druck eingedampft. Die so erhaltene ölige Base wird weiter umgesetzt. (Bei starker Verunreinigung empfiehlt sich eine Umkristallisation des Dihydrochlorids).

3.3. *N-Propargyl-N'-(3-trifluormethylphenyl)-piperazin-hydrochlorid*

Ein Gemisch, bestehend aus dem in der Vorstufe erhaltenen N-Propargyl-N'-(3-trifluormethylphenyl)-ethylendiamin und 20,2 g (0,2 mol) Triethylamin, wird in 500 ml absolutem Toluol auf 100°C erhitzt, während 18,8 g (0,1 mol) Dibromethan zugetropft werden. Es wird so lange erhitzt, bis gemäss chromatographischer Kontrolle die Reaktion beendet ist.

Nach beendeter Umsetzung wird die Reaktionslösung mit der berechneten Menge Natriumcarbonatlösung extrahiert. Nach dem Trocknen mit Natriumsulfat wird unter vermindertem Druck eingedampft und die zurückbleibende Base auf üblichem Weg mit einer stöchiometrischen Menge Chlorwasserstoffs zum Hydrochlorid umgesetzt.

Weisse Kristalle aus Acetonitril, Schmp. 218-219°C (Zers.).

*Beispiel 4*

*N-(3-Trifluormethyl-phenyl)-N'-propargyl-piperazin-hydrochlorid (nach Verfahren d)*

4.1. *N-(3-Trifluormethylphenyl)-piperazin-hydrochlorid*

71,4 g (0,4 mol) Bis-(2-chlorethyl)-amin-hydrochlorid und 64,4 g (0,4 mol) 3-Trifluormethylanilin werden in 500 ml n-Butanol suspendiert und unter Rückfluss erhitzt. Die im Verlauf der Reaktion auftretende saure Reaktion wird mit Natriumhydrogencarbonat abgepuffert. Das dabei entstehende Wasser wird azeotrop entfernt. Dieses Vorgehen wird nach jeweils 24 Stunden mit je 12,9 g (0,08 mol) 3-Trifluormethylanilin zweimal wiederholt.

Nach beendeter Reaktion wird die Suspension auf 10°C abgekühlt und die Kristalle abgesaugt. Das n-Butanol wird unter vermindertem Druck abdestilliert. Die abgesaugten Kristalle und der Destillationsrückstand werden in Wasser gelöst und mehrmals mit Ether extrahiert. Die Wasserphase wird mit Natriumcarbonat alkalisch gemacht und ausgiebig mit Ether extrahiert. Nach dem Trocknen mit Natriumsulfat wird unter vermindertem Druck eingedampft und die zurückbleibende Base auf üblichem Weg zum Hydrochlorid umgesetzt. Weisse Kristalle aus Ethanol. Schmp. 236-237°C (Zers.). Ausbeute: 65,2 g (61,1% d.Th.).

4.2. *N-(3-Trifluormethylphenyl)-N'-propargyl-piperazin-hydrochlorid*

3,2 g (0,012 mol) N-(3-Trifluormethylphenyl)-piperazin-hydrochlorid, 0,9 g (0,012 mol) Propargylchlorid und 2,5 g (0,024 mol) Natriumcarbonat

werden in 50 ml abs. Acetonitril unter Rühren auf 60°C erhitzt.

Im Verlauf von 15 Stunden werden weitere 0,34 g (0,0046 mol) Propargylchlorid und 0,5 g (0,0046 mol) Natriumcarbonat zugegeben.

Die anorganischen Salze werden durch Absaugen entfernt und die Reaktionslösung unter vermindertem Druck eingedampft. Der Destillationsrückstand wird in Toluol gelöst und das Produkt mit der berechneten Menge verdünnter Salzsäure extrahiert. Die Wasserphase wird mit Soda alkalisch gestellt und die Base mit Ether extrahiert. Nach dem Trocknen mit Natriumsulfat wird unter vermindertem Druck eingedampft und die Base auf üblichem Weg zum Hydrochlorid umgesetzt. Weisse Kristalle aus Acetonitril, Schmp. 218-219°C (Zers.). Ausbeute: 2,7 g (73,8% d.Th.).

Das Vorliegen dieser Verbindungen wurde durch Elementaranalyse und Spektrum bestätigt.

*Beispiel 5*

*N-(3-Trifluormethylphenyl)-N'-propargyl-piperazin-hydrochlorid (nach Verfahren e)*

5.1. *3-(Trifluormethylphenyl)-iminodiessigsäure*

Analog A. Reissert, Ber. dtsch. chem. Ges. 57, 993 (1924) bzw. N.B. Tien, J. Org. Chem. 23, 186 (1958) wird aus 3-(Trifluormethyl)-anilin die 3-(Trifluormethylphenyl)-iminodiessigsäure hergestellt.

5.2. *1-Propargyl-4-(3-trifluormethylphenyl)-piperazin-2,6-dion*

Die nach 5.1. gewonnene Verbindung wird in das Säureanhydrid übergeführt und dann mit Propargylamin zum 1-Propargyl-4-(3-trifluormethylphenyl)-piperazin-2,6-dion umgesetzt.

5.3. *Reduktion zum Endprodukt*

Das nach 5.2. erhaltene Dion wird mit Boran/Tetrahydrofuran oder Lithiumaluminiumhydrid in Ether jeweils bei Rückflusstemperatur zum Endprodukt reduziert [analog D.W. Henry, J. Het. Chem. 3, 503 (1966) bzw. G. Cignarella, J. Med. Chem. 7, 242 (1964)]. Das Hydrochlorid wird entsprechend Beispiel 1 erhalten, Schmp. 218-219°C (Zers.).

*Beispiel 6*

*N-(3-Trifluormethylphenyl)-N'-propargyl-piperazin-hydrochlorid (nach Verfahren f)*

6.1. *Propargyl-iminodiessigsäureanhydrid*

Aus Propargylamin wird mit Chloressigsäure zunächst die Propargyl-iminodiessigsäure hergestellt und diese in das Anhydrid übergeführt.

6.2. *4-Propargyl-1-(trifluormethylphenyl)-piperazin-2,6-dion*

Die Umsetzung des nach 6.1. erhaltenen Säureanhydrids mit 3-(Trifluormethyl)-anilin ergibt das oben genannte Dion.

6.3. *Reduktion zum Endprodukt*

Das nach 6.2. erhaltene Dion wird mit Boran/Tetrahydrofuran oder Lithiumaluminiumhydrid bei Temperaturen bis zur Rückflusstemperatur reduziert. Die Arbeitsweise entspricht der in Beispiel 5. Schmp. 218-219°C (Zers.).

*Beispiel 7*

*N-(3-Trifluormethylphenyl)-N'-propargyl-piperazin-hydrochlorid (nach Verfahren g)*

Die Kondensation des nach 3.2. erhältlichen N-Propargyl-N'-(3-trifluormethylphenyl)-ethylendiamins mit Oxalsäurediethylester ergibt 1-Propargyl-4-(3-trifluormethylphenyl)-piperazin-2,3-dion. Diese Verbindung wird mit Lithiumaluminiumhydrid oder Boran entsprechend Beispiel 5 zur Titelverbindung reduziert und aufgearbeitet. Weisse Kristalle, Schmp. 218-219°C (Zers.).

Statt mit HCl kann die freie Base auch z.B. mit HBr, $H_2SO_4$, $HNO_3$, $CH_3COOH$ und anderen Säuren in die entsprechenden Salze übergeführt werden.

B. Formulierungsbeispiele

1. *Tabletten*

| | |
|---|---|
| N-(3-Trifluormethylphenyl)-N'-propargylpiperazin-hydrochlorid | 75,0 mg |
| Maisstärke | 164,0 mg |
| sek. Calciumphosphat | 240,0 mg |
| Magnesiumstearat | 1,0 mg |
| | 480,0 mg |

Herstellung: Die einzelnen Bestandteilen werden intensiv miteinander vermischt und die Mischung in üblicher Weise granuliert. Das Granulat wird zu Tabletten von 480 mg Gewicht verpresst, von denen jede 75 mg Wirkstoff enthält.

2. *Gelatine-Kapseln*

Der Inhalt der Kapsel setzt sich wie folgt zusammen:

| | |
|---|---|
| N-(3-Trifluormethylphenyl)-N'-propargylpiperazin-hydrochlorid | 50,0 mg |
| Maisstärke | 200,0 mg |
| | 250,0 mg |

Herstellung: Die Bestandteile des Kapselinhalts werden intensiv vermischt und 250 mg-Portionen der Mischung werden in Gelatine-Kapseln geeigneter Grösse abgefüllt.
Jede Kapsel enthält 50 mg des Wirkstoffs.

3. *Depotdragées*

Kern:

| | |
|---|---|
| N-(3-Trifluormethylphenyl)-N'-propargylpiperazin · HCl | 60,0 mg |
| Carboxymethylcellulose (CMC) | 295,0 mg |
| Stearinsäure | 45,0 mg |
| Celluloseacetatphthalat (CAP) | 40,0 mg |
| | 440,0 mg |

Herstellung: Der Wirkstoff, die CMC und die Stearinsäure werrden intensiv gemischt und die Mischung in üblicher Weise granuliert, wobei man eine Lösung des CAP in 200 ml eines Gemisches aus Äthanol/Ätheracetat verwendet. Das Granulat wird dann zu 440 mg-Kernen verpresst, die in üblicher Weise mit einer zuckerhaltigen 5%igen Lösung von Polyvinylpyrrolidon in Wasser überzogen werden. Jedes Dragée enthält 60 mg Wirkstoff.

4. *Tabletten*

| | |
|---|---|
| N-(3-Trifluormethylphenyl)-N'-propargylpiperazin-hydrochlorid | 75,0 mg |
| Diazepam | 10,0 mg |
| Milchzucker | 164,0 mg |
| Maisstärke | 194,0 mg |
| kolloidale Kieselsäure | 14,0 mg |
| Polyvinylpyrrolidon | 6,0 mg |
| Magnesiumstearat | 2,0 mg |
| lösliche Stärke | 10,0 mg |
| | 475,0 mg |

Herstellung: Der Wirkstoff wird zusammen mit dem Milchzucker, der Maisstärke, der kolloidalen Kieselsäure und dem Polyvinylpyrrolidon nach intensiver Durchmischung in üblicher Weise granuliert, wobei man eine wässrige Lösung der löslichen Stärke verwendet. Das Granulat wird mit dem Magnesiumstearat gemischt und zu 1000 Tabletten von je 475 mg Gewicht verpresst, die je 75 mg des ersten und 10,0 mg des zweiten Wirkstoffs enthalten.

**Patentansprüche**

1. N-(3-Trifluormethylphenyl)-N'-propargylpiperazin der Formel I

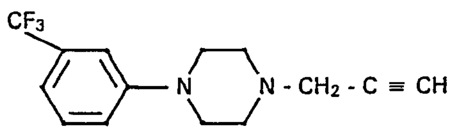 (I)

sowie dessen physiologisch verträgliche Säureadditionssalze.

2. Pharmazeutische Präparate, enthaltend als Wirkstoff die Verbindung der Formel I bzw. ein physiologisch verträgliches Säureadditionssalz davon in Kombination mit üblichen Hilfs- und/oder Trägerstoffen.

3. Verfahren zur Herstellung von pharmazeutischen Präparaten nach Anspruch 2, dadurch gekennzeichnet, dass man die Verbindung der Formel I bzw. deren physiologisch verträglichen Säureadditionssalze mit üblichen galenischen Hilfs- und/oder Trägerstoffen zu üblichen pharmazeutischen Anwendungsformen verarbeitet.

4. Verfahren zur Herstellung der Verbindung der Formel I bzw. deren physiologisch verträglichen Säureadditionssalzen, dadurch gekennzeichnet, dass man

a) eine Verbindung der allgemeinen Formel II

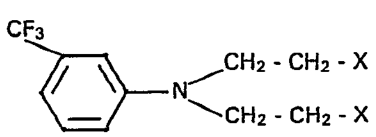 (II)

worin X eine leicht anionisch abspaltbare Gruppe bedeutet, mit Propargylamin der Formel III

$HC \equiv C - CH_2 - NH_2$ (III)

umsetzt oder dass man

b) eine Verbindung der allgemeinen Formel IV

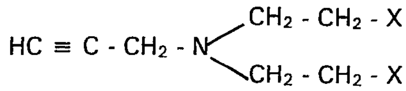 (IV)

worin X die obengenannten Bedeutungen hat, mit 3-Trifluormethylanilin der Formel V

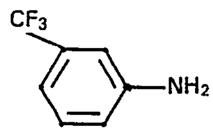 (V)

umsetzt oder dass man

c) die Verbindung der Formel VI

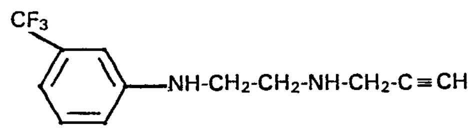 (VI)

mit einer Verbindung der allgemeinen Formel VII

$X - CH_2 - CH_2 - X$ (VII)

worin X die obengenannten Bedeutungen hat, umsetzt, oder dass man

d) in die Verbindung N-(3-Trifluormethylphenyl)-piperazin mittels einer Verbindung der allgemeinen Formel VIII

$HC \equiv C - CH_2 - X$ (VIII)

worin X die oben angegebene Bedeutung hat, die Propargylgruppe einführt oder dass man

e) ein Dion der Formel IX

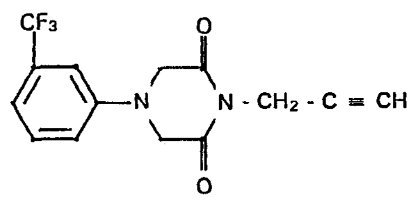 (IX)

mit reduzierenden Hydriden reduziert oder dass man

f) ein Dion der Formel X

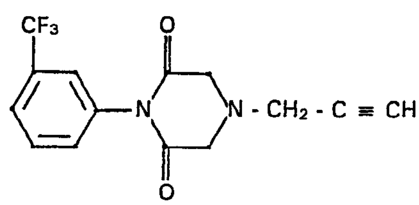 (X)

mit reduzierenden Hydriden reduziert oder dass man

g) ein Dion der Formel XI

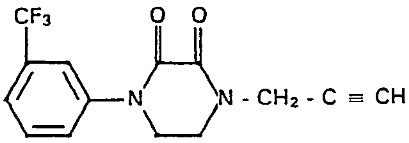

(XI)

mit reduzierenden Hydriden reduziert und dass man die nach a) bis g) erhaltene Verbindung gewünschtenfalls in Säureadditionssalze bzw. in das freie Amin der Formel I überführt, sofern sie als Salze erhalten wurde.

## Claims

1. N-(3-Trifluoromethylphenyl)-N'-propargylpiperazine of formula I

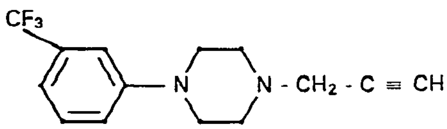

(I)

and the physiologically compatible acid addition salts thereof.

2. Pharmaceutical preparations containing as active substance the compound of formula I or a physiologically compatible acid addition salt thereof in combination with conventional excipients and/or carriers.

3. Process for preparing pharmaceutical preparations according to claim 2, characterised in that the compound of formula I or its physiologically compatible acid addition salts are processed with conventional galenic excipients and/or carriers into conventional pharmaceutical forms of administration.

4. Process for preparing the compound of general formula I or its physiologically compatible acid addition salts, characterised in that

a) a compound of general formula

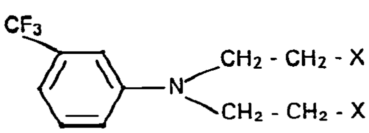

(II)

in which X represents a group which can be easily split off anionically, is reacted with propargylamine of formula III

$HC \equiv C - CH_2 - NH_2$ (III)

or in that

b) a compound of general formula IV

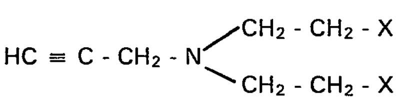

(IV)

in which X has the above-mentioned meanings, is reacted with 3-trifluoromethylaniline of formula V

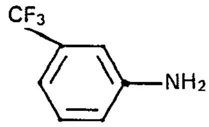

(V)

or in that

c) the compound of formula VI

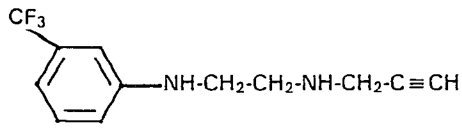

(VI)

is reacted with a compound of general formula VII

$X - CH_2 - CH_2 - X$ (VII)

in which X has the above-mentioned meanings, or in that

d) the propargyl group is introduced into the compound N-(3-trifluoromethylphenyl)-piperazine by means of a compound of general formula VIII

$HC \equiv C - CH_2 - X$ (VIII)

in which X has the above-mentioned meaning, or

e) a dione of formula IX

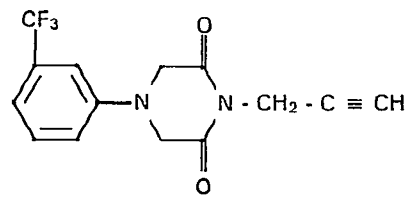

(IX)

is reduced with reducing hydrides, or

f) a dione of formula X

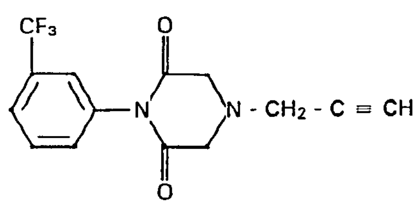

(X)

is reduced with reducing hydrides, or

g) a dione of formula XI

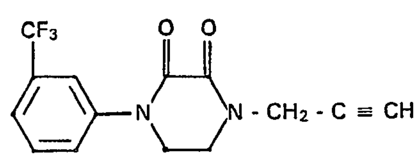

(XI),

is reduced with reducing hydrides and, if desired, the compound obtained according to a) to g) is converted into an acid addition salt or into the free amine of formula I, if it was obtained in the form of a salt.

**Revendications**

1. N-(3-trifluorométhylphényl)-N'-propargylpipérazine de formule I

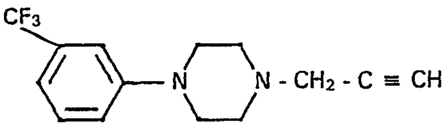 (I)

ainsi que ses sels d'addition d'acides physiologiquement supportables.

2. Préparations pharmaceutiques contenant, en tant que substance active, le composé de formule I ou un sel d'addition d'acide physiologiquement supportable de celui-ci, en combinaison avec des adjuvants et/ou excipients usuels.

3. Procédé pour la fabrication de préparations pharmaceutiques selon la revendication 2, caractérisé en ce que l'on transforme le composé de formule I ou ses sels d'addition d'acides physiologiquement supportables, au moyen d'adjuvants et/ou excipients galéniques usuels, en des formes d'utilisation pharmaceutiques usuelles.

4. Procédé pour la préparation du composé de formule I ou de ses sels d'addition d'acides physiologiquement supportables, caractérisé en ce que:

a) on fait réagir un composé de formule générale II:

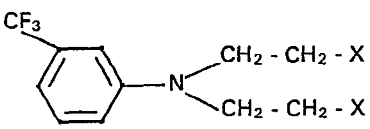 (II)

dans laquelle X représente un groupe facilement clivable à l'état anionique, avec la propargylamine de formule III:

$HC \equiv C - CH_2 - NH_2$ (III)

ou en ce que:

b) on fait réagir un composé de formule générale IV:

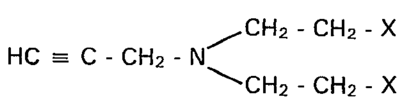 (IV)

dans laquelle X a les significations mentionnées plus haut, avec la 3-trifluorométhylaniline de formule V:

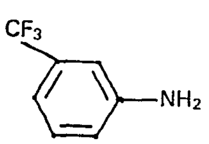 (V)

ou en ce que:

c) on fait réagir le composé de formule VI:

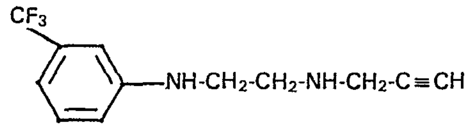 (VI)

avec un composé de formule générale VII:

$X - CH_2 - CH_2 - X$ (VII)

dans laquelle X a les significations mentionnées plus haut, ou en ce que:

d) on introduit le groupe propargyle dans le composé N-(3-trifluorométhylphényl)-pipérazine au moyen d'un composé de formule générale VIII:

$HC \equiv C - CH_2 - X$ (VIII)

dans laquelle X a la signification mentionnée plus haut, ou en ce que:

e) on réduit une dione de formule IX:

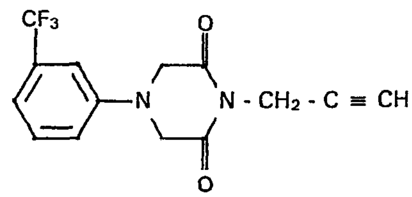 (IX)

au moyen d'hydrures réducteurs, ou en ce que:

f) on réduit une dione de formule X:

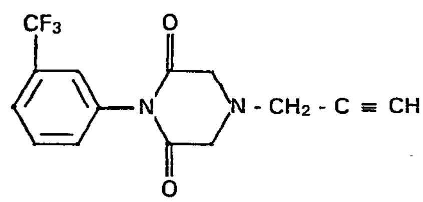 (X)

au moyen d'hydrures réducteurs, ou en ce que:

g) on réduit une dione de formule XI:

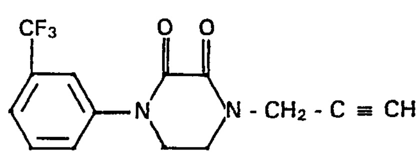 (XI)

au moyen d'hydrures réducteurs,

et en ce que, si on le désire, on transforme le composé obtenu selon a) à g) en des sels d'addition d'acides ou en l'amine libre de formule I, dans la mesure ou il a été obtenu sous forme de sel.